# EUROPEAN PATENT APPLICATION

(11) **EP 4 717 287 A1**
(43) Date of publication of application: **01.04.2026**
(21) Application number: 24203531.9
(22) Date of filing: 30.09.2024
(51) Int. Cl.: A61M 5/142, A61M 39/10, A61M 39/08

(54) **A CONNECTOR FOR POSITIONING A PUMP HOSE SEGMENT**

(71) Applicant: Fresenius Medical Care Deutschland GmbH, 61352 Bad Homburg (DE)
(72) Inventor: CAVALLI, Riccardo, 61352 Bad Homburg (DE); URBAN, Martin, 61352 Bad Homburg (DE); VENERONI, Alain, 61352 Bad Homburg (DE)
(74) Representative: Lorenz Seidler Gossel Part. mbB

(57) **Abstract**

The present invention provides a connector for positioning a pump hose segment in a pump bed of a medical peristaltic pump, characterized in that an optical data code, in particular a one-dimensional or two-dimensional barcode, is arranged on the connector.

## Description

The present invention relates to a connector for connecting a pump hose segment to a pump bed of a medical peristaltic pump, a corresponding pump hose set, a medical peristaltic pump, a medical device and a method for manufacturing the pump hose set.

The connector may in particular be used with a rotor pump where the pump hose segment is inserted into the pump bed in arcuate form. As it will be apparent from the description, the connector may also be used in conjunction with a linear peristaltic pump, such as a finger pump. In such a finger pump the pump hose segment on which the actors of the finger pump act, may be linear instead of forming a loop or in part a loop.

Peristaltic pumps are often used in medical devices such as blood treatment machines for pumping medical liquids, and in particular for pumping blood, dialysis fluid and/or priming fluid in a blood treatment machine such as a dialysis machine. Other medical fluids may be an anticoagulation fluid such as a citrate solution or heparin solution, or medical fluids such as a calcium ion solution, physiological sodium chloride solution. Accordingly, the connector as described within the description below may be configured to be used to pump one of these medical fluids, in particular may be connected to a tubing set that is connected to a source of one of these medical fluids.

Such a connector is known from EP 2 976 128 A1, showing a connector where the fluid paths corresponding to a first and second end of the pump hose cross in the connector.

Document EP 4 180 069 A1 shows a pump hose segment to be used with such a connector, wherein the pump hose segment comprises an information storage means comprising information pertaining to at least one individual characteristic of the pump hose segment. The corresponding medical pump comprises reading means for obtaining the information from the information storage means, and processing means adapted to adjust the pumping algorithm of the pump engine based on the information. In the embodiment, the information storage means is an RFID chip. The information may in particular be a geometrical dimension of the pump hose segment such as the inner diameter of the pump hose segment, wall thickness of the pump hose segment, and/or overall length of the pump hose segment measured, which has an influence on the flow rate of the pump hose segment, and which is individually measured during production for the pump hose segment and individually stored on the storage means of the segment.

RFID chips are however expensive and difficult to sterilize. Document EP 4 180 069 A1 mentions that the information storage means could equally an optical marking, such as a laser marking.

However, the inventors of the present invention have found out that if an optical marking is used in the system as shown in Document EP 4 180 069 A1, it is difficult to reliably read out the corresponding information. This applies in particular during the operation of the pump.

The object of the present invention is therefore to provide an improved system for providing data about a tubing system, by providing a corresponding connector, pump hose set, medical peristaltic pump, medical device and method for manufacturing the pump hose set.

This object is solved by the subject matter of the independent claims. Preferred embodiments of the present invention are the subject matter of the dependent claims.

The present invention provides a connector for positioning a pump hose segment in a pump bed of a medical peristaltic pump, in particular in arcuate form, wherein an optical data code, in particular a one-dimensional or two-dimensional barcode, a QR Code or a data matrix code is arranged on the connector.

Therefore, according to the present invention, the optical data code is not arranged on or at a hose segment, such as the pump hose segment, but on the connector used for connecting the pump hose segment to the medical peristaltic pump and positioning it in the pump bed of the medical peristaltic pump.

In particular for a peristaltic pump the actors, for example one or more rollers or one or more fingers, may act on the pump hose segment by pressing, thereby closing, the pump hose segment against the pump bed. When the pump actor does not press against the pump hose segment, the pump hose segment may be in no physical contact to the pump bed at least in part but being positioned in the pump bed by rhe connector being connected to the medical peristaltic pump..

Placing the optical data code on the connector rather than on the pump hose segment as in EP 4 180 069 A1 may have several significant advantages which may be all or some of them realized at the same time:
First of all, the connector is very well defined (actually better defined than most other components) in its position and firmly fixed. This allows for a reliable interface definition to an optical sensor integrated in the pump bed for reading the optical data code and there are little tolerances with respect to the misalignment of the optical data code and the optical sensor. In contrast, the pump hose segment is a flexible element that will change its position relative to the connector and therefore relative to the medical peristaltic pump when forces act on it, such as the forces acting on the pump hose for inserting it into the pump bed, or due to the rotor of the pump acting on the pump hose segment. In particular, once the pressing elements of the rotor are acting on the pump hose, the pump hose is deformed. This deformation will also depend on the position of the rotor. Further, during operation of the pump, the pump hose segment will even move regularly with the rotation of the rotor. These problems are avoided by placing the optical data code on the connector rather than on the pump hose segment.

Another advantage of placing the optical data code on the connector rather than on the tubing segment is that the tubing segment is gripped by the medical staff when it is attached to the pump bed, which could damage the code if it were attached to the tubing, whereas the connector is only gripped at defined positions, e.g. from the side, leaving sufficient space to place the code at a position that is not touched during the handling of the connector.

Another advantage of placing the optical data code on the connector is that the shape of the connector can be adapted to accommodate the optical data code and/or improve the readability of the data code, such as by avoiding stray light and/or avoiding that the data code is inadvertently touch and damaged.

Further, the medical peristaltic pump and, therefore, the connector for the pump hose segment is present in every tubing set also in case different variants of the tubing sets may be used with the medical device comprising the medical peristaltic pump.

Further, the medical peristaltic pump is a complex and crucial building block which may be used in different variants of the medical device and also remains often unchanged even if the medical device is further developed. Thus, the interface between connector and sensor remains regularly unchanged and is not to be modified/adapted in case the device is continued to be developed.

Further, the fluid that is pumped may vary, e.g. the blood that is pumped may have varying color dependent on the constitution of the blood or the same pump may once be used to pump a transparent medical fluid and in another situation may be used to pump blood, e.g. during different treatment phases such as priming, treatment, reinfusion or emptying. As the pump hose segment may be at least in part transparent, the optical appearance of the code may vary and, therefore, imposes a challenge for a camera to correctly detect the code. The connector may be non-transparent at least in the area where the code is positioned and may have a certain color, e.g. red, blue, white, green. As this is a predefined color the optical system to detect the code may be optimized for recognizing the code for that specific color.

Further, as described above, the pump hose segment is in continuous interaction with the rotor of the pump during pumping action which may lead to damage of the code, e.g. by abrasion of the color used for printing the code or of a sticker on which the code is provided on the hose or by wear through continuous squeezing i.e. closing and opening of the pump hose. The code on the connector may however be non-affected by the pumping action. The code may be kept in a predefined distance from a surface of the detection system when positioned in the pump. The area where the code is positioned on the connector may be recessed in comparison to an abutting structure of the connector, wherein the abutting structure may be a structure with which the connector is abutting against a surface of the pump.

Finally, in a usual medical device, the area of the medical peristaltic pump is an area that needs to be cleaned carefully anyway after every treatment. Accordingly, the machine side of the interface between sensor and connector is automatically subject to regular cleaning and, accordingly, a deterioration of the optical system is of low risk as a sensor window is also regularly cleaned.

According to an embodiment of the present invention, instead of using an active device as a data element, such as an RFID chip or other active element having a broadcasting function, an optical data code is used that is not an active device, but a passive element containing the data as a permanent optical code for reading by an optical sensor. This provides both a robust and inexpensive solution for applying data to the connector.

In an embodiment, the optical data code is provided on a part of the connector that is immobile with respect to a main body of the connector. Thereby, it is assured that the position of the optical data code is well defined relative to the peristaltic medical pump once the main body of the connector is connected to the peristaltic medical pump, and its position cannot be changed even when forces act on the pump hose and via the pump hose on the connector, such as during insertion of the pump hose or by the action of the pump rotor onto the pump segment.

The present invention can be applied to every connector for positioning at least one pump hose segment in a pump bed of a medical peristaltic pump. The pump bed may be in arcuate form, i.e. such that the pump hose segment forms a pump loop arranged within an arcuate pump bed of the medical peristaltic pump, with a rotor of the medical peristaltic pump acting on the pump loop for pumping. The pump bed may be in a linear form, i.e. such that a pump hose segment forms a linear pump hose segment within a linear pump bed of the medical peristaltic pump, with finger like actors of the medical peristaltic pump acting on the linear pump hose for pumping.

Only one end of the pump hose segment may be connected to the connector or two ends of the pump hose segment may be connected to the connector.

Preferred connectors for which the present invention can be applied with particular advantages will be described in the following:
According to an embodiment of the present invention, the connector comprises a first fluid path associated with a first end of the pump hose segment and a first pump hose receiving section associated with the first fluid path for receiving the first end of the pump hose segment.

In particular, the first end of the pump hose segment may be connected to the first pump hose receiving section for fluidly coupling the first fluid path of the connector with the pump hose segment. For example, the first end of the pump hose segment may be inserted into the first pump hose receiving section and/or glued to the first pump hose receiving section.

According to an embodiment of the present invention, the connector comprises in addition to the first fluid path a second fluid path associated with a second end of the pump hose segment and a second pump hose receiving section associated with the second fluid path for receiving the second end of the pump hose segment.

In particular, the second end of the pump hose segment may be connected to the second pump hose receiving section for fluidly coupling the second fluid path of the connector with the pump hose segment. For example, the second end of the pump hose segment may be inserted into the second pump hose receiving section and/or glued to the second pump hose receiving section.

Other options for connecting the pump hose segment to the pump hose receiving sections are clicking or screwing.

In an embodiment of the present invention, the optical data code is provided on an element of the connector that is immobile relative to the first and/or second pump hose receiving section. In particular, the optical data code is provided on an element of the connector that does not move relative to the first and/or second pump hose receiving section when forces act on the pump hose segment, e.g. when the pump hose segment is inserted into the pump bed or the pump rotor acts on the pump hose segment.

According to an embodiment of the present invention, the connector comprises a first connecting hose receiving section associated with the first fluid path for receiving a first connecting hose segment.

In particular, the first connecting hose segment may be connected to the first connecting hose receiving section for fluidly coupling the first fluid path of the connector with the first connecting hose segment. For example, the first connecting hose segment may be inserted into the first connecting hose receiving section and/or glued to the first connecting hose receiving section.

According to an embodiment of the present invention, the connector comprises a second connecting hose receiving section associated with the second fluid path for receiving a second connecting hose segment.

In particular, the second connecting hose segment may be connected to the second connecting hose receiving section for fluidly coupling the second fluid path of the connector with the second connecting hose segment. For example, the second connecting hose segment may be inserted into the second connecting hose receiving section and/or glued to the second connecting hose receiving section.

According to an embodiment of the present invention, the connector may be configured to establish a first fluid connection between the first connecting hose segment and the first end of the pump hose segment via the first fluid path and a second fluid connection between the second connecting hose segment and the second end of the pump hose segment.

According to an embodiment of the present invention, the connector may be configured to establish fluid connections between the first connecting hose segment, the second connecting hose segment and the pump hose segment such that when inserted in the pump bed of a peristaltic pump, fluid can be pumped from the first connecting hose segment via the first fluid path, the pump hose segment and the second fluid path to the second connecting hose segment and/or vice versa.

According to an embodiment of the present invention, the first fluid path and the second fluid path cross in the connector. Advantages of such a configuration are described in EP 2 976 128 A1.

According to a first embodiment of the present invention, the connector comprises an integrally formed connector part comprising the first and, optionally, the second fluid path.

According to an embodiment of the present invention, the connector and/or the connector part is formed by injection molding as a single, integral piece.

In an embodiment of the present invention, the optical data code is provided on an element of the connector that is immobile relative to the integrally formed connector part and in particular is provided on the integrally formed connector part. The optical data code may also be provided on a separate part of the connector that is fixedly connected to the integrally formed connector part. Fixedly connected may have the meaning of not being movable relative to the integrally formed connector part. However, fixedly connected does not have the meaning that it may only be separated by destructive forces. For example, the separate part of the connector and the integrally formed connector part may be two separate elements that are joint e.g. by clicking or sliding into respective fixation means provided on the two separate elements.

According to a second embodiment of the present invention, the connector comprises a first connector part comprising the first fluid path and a second connector part comprising the second fluid path.

According to an embodiment of the present invention, each connector part or the connector is formed by injection molding as a single, integral piece.

In an embodiment of the present invention, the optical data code is provided on an element of the connector that is immobile relative to first connector part and/or the second connector part, and in particular is provided on the first connector part or the second connector part.

The expression "immobile relative to" has the meaning that the element on which the data code is provided may only move together with the connector part to which it is immobile, wherein moving not only means moving in space but also means changing its shape e.g. by squeezing, stretching. This, in particular, may apply when the connector is positioned in the pump.

The element on which the data code is provided may be basically not flexible, in particular not squeezable or stretchable.

The element on which the data code is provided may be at least in part be made of a formstable material, in particular plastic such as polypropylene or polycarbonate. The data code may be provided on the part made of the formstable material or is provided on a part of the connector made of a non-formstable material with the part of the connector made of a non-formstable material being attached to the part made of the formstable material such that the position of code is fixed relative to the formstable material. An example of such a structure is a code provided on a flexible membrane as non-formstable material, but the membrane is fixed to e.g. a part made of a formstable material surrounding at least in part the membrane so that the membrane is fixed on its position or immobile relative to the part made of formstable material.

The part made of the formstable material may be connected to a connection part of the connector or may comprise a connection part that is configured to be connected to a corresponding structure of the pump such that the connector is firmly fixed to the structure of the pump, in particular during operation of the pump.

During operation of the pump a movement, in particular any movement, of the code may be smaller than the movement of a pump loop connected to the connector. In particular a movement of the code towards and away from the detection system and or in a direction vertical to a plane of the code may be smaller than the distance by which any surface, in particular a surface of the pump hose segment oriented towards the pump bed, is displaced due to the pumping operation.

According to an embodiment of the present invention, the optical data code is arranged on a functional element of the connector, i. e. an element that is provided in view of the functionality of the connector, such that there is no need to provide any additional elements on the connector for placing the optical data code. The surface on which the optical data code is arranged may be a non-flat surface or a flat surface. Such a functional element of the connector may be a part of the connector forming a fluid path or an element for exerting a manual force to insert the connector in the pump or a fixing structure to attach the connector to the pump or a pressure measuring chamber.

According to an embodiment of the present invention, the optical data code is arranged on an outer surface of the connector, in particular an element of the first or second fluid path. The elements of the first or second fluid path may be the functional elements of the connector having the best suited surfaces for carrying the optical data code.

According to an embodiment of the present invention, the optical data code is arranged on the first and/or second pump hose receiving section.

The inventors of the present invention have realized that the pump hose receiving sections are suitable for placing the optical data code. For example, the pump hose receiving sections are very clearly defined in their position, provide sufficient space for placing the optical data code, and are positioned when connected to the pump in a position that allows easy access by the optical sensor of the pump and sufficient space in the pump to position the optical sensor.

According to an embodiment of the present invention, an inner and/or outer diameter of the first and/or second pump hose receiving section is larger than an inner and/or outer diameter of the first and/or second connecting hose receiving section.

The inner diameter of the first and/or second pump hose receiving section may be larger than the inner diameter of the first and/or second connecting hose receiving section for reasons of improved fluid flow and pumping action because the pump hose segment usually has a larger outer diameter than the connecting hose sections. The pump hose segment may have a higher flexibility for being compressed than the connecting hose. This supports the action of actors e.g. rollers or fingers of a peristaltic pump.

Consequently, the outer diameter of the first and/or second pump hose receiving section is usually larger than the outer diameter of the first and/or second connecting hose receiving section, and therefore the first and/or second pump hose receiving section provides more space than the first and/or second connecting hose receiving section for placing the optical data code.

In alternative configurations, the optical data code is provided on a part of the connector different from the first and/or second pump hose receiving section.

In an embodiment, the optical data code may be provided on the first and/or second connecting hose receiving section.

In an embodiment, the optical data code may be provided on an element specifically provided for placing the data code. The element may in particular have a surface for placing the data code on the bottom side of the connector. The element may protrude towards the bottom side from one of the fluid paths and/or other functional elements of the connector.

In an embodiment, the element may have a surface portion for placing the optical data code that has a size of at least 5mm x 5 mm and/or a size smaller than 20 mm x 20 mm.

In an embodiment, the optical data code may be provided on a plate-like element, in particular a plate-like element specifically provided on the connector for placing the optical data code, or a plate-like element extending from a functional element and in particular connecting functional elements of the connector such as hose receiving sections or another functional element described above .

The plate-like element may be attached to at least one of the hose receiving sections and/or may extend in a plane that is perpendicular to the axis of rotating of the medical peristaltic pump when the connector is connected to the medical peristaltic pump.

In an embodiment, the optical data code may be provided on an element having a flat surface. This is in particular possible if the element is specifically provided and/or shaped for carrying the optical data code.

In an embodiment, the optical data code may be provided on the bottom side of the connector at a position where the fluid paths cross inside the connector. The outer surface of the fluid path arranged on the bottom side may comprise an enlarged and/or flattened section at this position to place the optical data code. The enlarged and/or flattened section may for example have a flat surface, in particular a flat surface extending in a plane that is perpendicular to the axis of rotating of the medical peristaltic pump when the connector is connected to the medical peristaltic pump. Such an orientation may allow for a reduction of the movement of the connector and, therewith, the code in a direction of the axis of the rotation during pumping action. This may have the advantage that the focus depth of the detection system may be kept small and, therefore, a cheaper detection system may be used.

Preferably, the optical data code is not provided on a side surface of the connector. However, in a possible embodiment, the optical data code is provided on a side surface of the connector.

According to an embodiment of the present invention, the optical data code is formed by laserwriting. In other embodiments the optical data code is provided by printing or by a sticker with the optical code on it fixed to the connector. The sticker may for example be made of plastic or paper.

Laser writing (also sometime call laser marking) is simple, efficient, and made without any sort of ink. Further, the content of the optical data code can be easily generated without any special setup. Further, for laser writing, there is no need of any kind of consumable or spare parts.

It is not toxic (no ink is used) and is also insensible against sterilization.

According to an embodiment of the present invention, the optical data code is written by at least two consecutive passes of laserwriting. Preferably, it is written by at least three consecutive passes of laserwriting.

The inventors of the present invention have realized that the contrast of the laserwriting may improve with the number of passes.

In an embodiment of the present invention, the connector has a base color that is white, red or blue. The inventors of the present invention have realized that these colors provide a good contrast of the laser writing. However, the connector may also have a base color that is transparent.

Preferably, the color of the optical data code is selected depending on the base color of the connector to provide a good contrast.

For example, the connector may have a red basic color and a white optical data code, or a white basic color and a black optical data code. A further alternative is a blue as base color and preferably white for the optical data matrix.

In an embodiment of the present invention, the medical device, in particular the medical peristaltic pump or the pump bed, comprises a lighting unit for lighting the optical data code when the connector is connected to the medical peristaltic pump for reading the optical data code.

The medical device, in particular the medical peristaltic pump or the pump bed, may comprise an optical sensor for reading the optical data code.

In this respect, we note that any element or feature that is realized in the embodiments described in the following as part of the pump or as located in the pump bed, such as the lighting unit and/or sensor, may also be positioned separately from the pump or pump bed in the machine housing of the medical device so that there is no direct physical contact between them.

In an embodiment of the present invention, the lighting unit emits colored light. In an embodiment of the present invention, the lighting unit emits colored light having a complementary color with respect to the base color of the connector to reach a better contrast.

In an embodiment of the present invention, the housing of the pump comprises, at the position of the optical sensor and/or the lighting unit, a raised frame protruding outwards towards the optical data code. This allows the light to fall directly onto the optical data code and avoids side or top and bottom shadows or stray light.

In particular, the raised frame may partially and/or completely surround an optical window provided in the housing of the pump, the optical sensor and/or the lighting unit arranged behind the window. Alternatively, the raised frame may be provided directly on the optical sensor and/or the lighting unit.

In an embodiment of the present invention, the optical sensor and/or the lighting unit is arranged behind a window provided in the housing of the medical peristaltic pump.

In an embodiment, the window in provided flush with the surface of the housing surrounding the window. This is easier to clean.

In an embodiment of the present invention, the connector comprises a raised frame protruding outwards towards the sensor and/or the lighting unit and partially and/or completely surrounding the optical data code. This avoids stray light shining in from the sides. Such a raised frame will have the additional advantage that it avoids medical personnel from inadvertently touching the data code and thereby damaging or spoiling the data code.

In an embodiment of the present invention, both the connector and the housing of the pump each comprise a raised frame protruding outwards towards each other when the connector is connected to the pump.

The frame or frames may be provided such that the connector and the housing of the pump abut on each other around the optical data code, such that there is no slit provided between the connector and the housing, or nearly touch each other such than only a small slit remains, thereby effectively blocking stray light.

In an embodiment, the connector and the housing of the pump are configured such that a surface of the connector provided with the optical data code abuts with a window provided in the housing for the optical sensor and/or the lighting unit.

In an embodiment of the present invention, a door is provided on the pump covering the connector that reduces stray light. The door may also cover the rotor and/or the pump bed.

The distance between the optical sensor and/or the lighting unit and the data code provided on the connector is preferably between 5 cm and 15 cm. Therefore, preferably, the optical sensor and/or the lighting unit is provided inside the housing of the pump at a distance to a window provided in the housing.

According to an embodiment of the present invention, the optical data code is formed on a no-flat surface of the connector. This non-flat surface may be on the rounded hose receiving section. Therefore, no extra surface has to be provided on the connector.

In another embodiment, an extra surface is provided for positioning the optical data code. If the extra surface is provided, the extra surface is preferably a flat surface.

According to an embodiment of the present invention, the optical data code is formed on a flat surface of the connector.

According to an embodiment of the present invention, a distortion of the surface in an image of an optical sensor reading the optical data code is compensated by a distortion of the optical data code. Therefore, the distortion of the optical code provided on the non-flat surface can compensate for the 3D shape of the non-flat surface such that in the image of the optical sensor reading the optical data code the optical data code is not distorted, e.g. such that e.g. the data elements of the optical data code such as lines or dots have the same size and/or shape.

According to an embodiment of the present invention, individual bits of the Code in a center area of the code are differently designed, in particular with a smaller area, than in an edge area of the code, in particular with a gradually increasing area from the center to the edge

Preferably, the optical data code is not provided on a side surface of the connector.

According to an embodiment of the present invention, the optical data code is arranged on a bottom side of the connector facing a receiving area of the medical peristaltic pump. First of all, the receiving area of the medical peristaltic pump is a particularly convenient place to arrange the optical sensor for reading the optical code, in particular in view of the available space and the fact that this area is regularly cleaned, anyway. Further, the relative position between the optical sensor and the optical data code is well defined. Finally, the influence of stray light on the reading is reduced.

As indicated above, the bottom side of the connector is the side of the connector facing a receiving area of the medical peristaltic pump. It is called bottom side because it is, relative to the direction of the axis of the rotor of the medical peristaltic pump, the side of the connector facing the medical peristaltic pump, while the opposite side, which is called upper side in the context of the present invention, faces away from the medical peristaltic pump. This definition is independent from the absolute direction of the axis of the rotor of the medical peristaltic pump, which in operation and/or when part of a medical device, may extend in any direction, and may in particular extend horizontally.

According to an embodiment of the present invention, the connector comprises besides the fixation element or elements, tube receiving section, the optical data code at least one, but optionally more, functional elements of the group comprising a abutment element, a anticoagulation tube receiving section with an optionally smaller opening diameter than the other tube receiving sections, a flexible membrane for a pressure measurement.

According to an embodiment of the present invention, the connector comprises at least one abutment element cooperating with at least one ejector of the medical peristaltic pump arranged on the bottom side of the connector for ejecting the connector. The at least one ejector of the medical peristaltic pump may in particular comprise an ejector pin that abuts with and presses against the abutment element of the connector to eject the connector from its connected state with the medical peristaltic pump. The abutment element is placed on the bottom side of the connector for this purpose.

Therefore, the abutment element thereby defines one of the sides of the connector as its bottom side.

In an embodiment of the present invention, the optical data code is provided on the same side of the connector as the at least one abutment element.

According to an embodiment of the present invention, in plan view, the connector has a first part comprising the first pump hose receiving section and a second part comprising the second pump hose receiving section, wherein the optical data code is arranged in one of the parts and the at least one abutment element in the other, remaining part. In an embodiment, the parts may each form one half of the connector in plan view.

Therefore, there is sufficient space within the rejecting area of the medical peristaltic pump for the ejector and for the optical sensor.

According to an embodiment of the present invention, the abutment element is arranged in an area of the connector extending between the pump hose receiving section and a connecting hose receiving section arranged in the corresponding part.

The connector of the present invention is not particularly limited with respect to the way in which the connector is attachable to the medical peristaltic pump and/or the medical device.

According to embodiments of the present invention, the connector comprises a connection part by which it is connectable to a corresponding structure of the medical device and/or the pump, in particular a receiving structure receiving the connection part.

According to embodiments of the present invention, the connector may be attachable to the medical peristaltic pump and/or the medical device by one or more of the following: a clip-connection, a form-fitting connection, a force-fitting connection, mechanical clamping elements, a vacuum fixation by suction applied onto a surface of the cassette coupled to a surface of the medical peristaltic pump and/or the medical device. For example, the medical peristaltic pump and/or the medical device may comprise clip-connection counter elements, form-fitting counter elements and/or force-fitting counter elements cooperating with corresponding connection elements of the connector. Further, the medical peristaltic pump and/or the medical device may comprise mechanical clamping elements or a vacuum fixation system comprising a vacuum source and a coupling surface for the connector, wherein the vacuum source is connected or connectable with a space between the coupling surface and the connector.

According to an embodiment of the present invention, the connector comprises at least one clip-element for being connected to the medical peristaltic pump and/or the medical device by a clip-connection. The connector may in particular comprise at least two clip-elements, such as clip-arms.

According to an embodiment of the present invention, the connector is configured to be arranged in an opening of the pump bed. Thereby, the first and the second fluid flow paths of the connector extend out of the pump bed at a position below the upper edge of the pump bed.

According to an embodiment of the present invention, the connector is configured to be clipped into the opening of the pump bed and/or comprises two clip elements arranged on two sides, optionally on opposite sides of the connector for clipping to mating connecting surfaces of the pump bed.

According to an embodiment of the present invention, a first clip element has a rounded shape and a second clip element has an angled shape.

According to an embodiment of the present invention, one of the clip elements, preferably a first clip element having a rounded shape, is provided with an abutment element for connecting to an ejection pin of the medical peristaltic pump.

According to an embodiment of the present invention, the optical data code is preferably arranged on a side of the connector comprising the second clip element.

According to an embodiment of the present invention, a first clip element is arranged on a side of the first pump hose receiving section and a second clip element is arranged on a side of the second pump hose receiving section.

According to a first embodiment of the present invention, the clip elements are integrally formed as parts of a connector part comprising the first and the second fluid path, and in particular integrally formed as parts of a main body of the connector, e.g. by injection molding.

According to a second embodiment of the present invention, the clip elements are separately formed as at least one separate element connected to a connector part comprising the first and the second fluid path. For example, the connector may comprise a first connector part comprising the clip elements and a second connector part comprising the first and the second fluid path, which are separately formed and attached to each other, e.g. by gluing, welding or a snap-, form- or force-fit connection.

According to an embodiment of the present invention, a main body of the connector is formed by or comprises two tubular fluid path elements comprising the first and a second fluid path, wherein each of the tubular fluid path elements comprises a pump hose receiving section on one end and a connecting hose receiving section on the opposite end.

According to an embodiment of the present invention, the connector further comprises a third fluid path connected to the first or the second fluid path and a third connecting hose receiving section associated with the third fluid path for connecting a third connecting hose to the first or the second fluid path via the third fluid path.

According to an embodiment of the present invention, the third fluid path is provided by a third tubular fluid path element connected to the first or second tubular fluid path element with one end and comprising the third connecting hose receiving section on its other end.

According to an embodiment of the present invention, the third tubular fluid path element extends in parallel to the second or first tubular fluid path element, preferably with a distance therebetween.

According to an embodiment of the present invention, the connector comprises at least one pressure sensor interface configured to be connected to a pressure transducer.

According to an embodiment of the present invention, the pressure sensor interface comprises a flexible membrane covering an internal cavity of the connector, the internal cavity fluidly connected to the first or the second fluid path.

According to an embodiment of the present invention, the connector is configured as a cassette comprising at least one functional element such as a pressure sensor interface, fluid control element and/or fluid degassing element.

According to an embodiment of the present invention, the connector is configured as a cassette that connects at least a first and a second pump hose segment to a first and a second pump bed in arcuate form.

According to a preferred embodiment of the present invention, the optical data code is provided on a tubular element of the connector that comprises, on one end, a pump hose receiving section, or on an element of the connector that is directly connected to and/or arranged at such a tubular element. This is preferred even if the connector is configured as a cassette.

However, in case that the connector is configured as a cassette, the optical data code may also be provided on a bottom or upper side of a part of the cassette extending in a plane and connected to a coupling surface of the medical device.

According to an embodiment of the present invention, the cassette may comprise a hard part in which fluid paths are recessed, and a flexible plastic film that is provided on one side of the hard part and covers the fluid paths. The cassette may be configured to the connected via the flexible film to a coupling surface of the medical device. In such an embodiment, the optical data code may be arranged on the flexible film and/or on the hard part.

The present invention can however also be used with connectors which are not configured as a cassette.

According to an embodiment of the present invention, the connector may in particular have no fluid paths branching off from the first and second fluid path, or only a single fluid path branching off from the first or the second fluid path. If there is a fluid path branching off from the first or the second fluid path, it may be a fluid path for a heparin line.

According to an embodiment of the present invention, the optical data code may be provided on a surface of the connector that is arranged on an axis of the connector defined by a pump hose receiving section and the corresponding connecting hose receiving section, or in an area between two axes of the connector each defined by a pump hose receiving section and the corresponding connecting hose receiving section.

In an embodiment of the present invention, the connector is a connector as shown in EP 2 976 128 A1, in EP application 23 166 082.0, in EP application 23 187 144.3, in EP application 23 187 154.2, in PCT/EP2024/070838 or in PCT/EP2024/070879, the disclosure of which is herewith incorporated in its entirety.

According to an embodiment of the present invention, the connector is sterile and/or provided in sterilized packaging. In particular, the connector and/or its packaging may be sterilized by the application of heat, of gas and/or of UV rays.

The present invention further comprises a pump hose set comprising a connector as discussed above and a pump hose segment connected to the connector and configured for insertion into a pump bed of a medical peristaltic pump in arcuate form.

The pump hose set has the same advantages as already discussed above with respect to the connector of the present invention. Further, the pump hose segment and/or the connector of the set may be configured as discussed above and the following with respect to the inventive connector.

According to an embodiment of the present invention, the pump hose set further comprises two connecting hose segments connected to the connector.

According to an embodiment of the present invention, the connecting hose segments are provided with connection elements at their distal ends.

According to an embodiment of the present invention, the pump hose set is a blood tubing set comprising a connection element for connecting to a dialyzer provided on one of the connecting hose segments, preferably an outlet connecting hose segment.

According to an embodiment of the present invention, the pump hose set is sterile and/or provided in sterilized packaging. In an embodiment, the pump hose set and/or its packaging may be sterilized by the application of heat, gas and/or UV rays.

According to an embodiment of the present invention, data indicative of the specific properties of the pump hose set and in particular the specific pump hose segment and/or connecting hose segments of the pump hose set may be stored in the optical data code of the connector used in the set. Therefore, the data may be specifically determined for each pump hose set, in particular based on a measurement and/or calibration performed on the pump hose set, before they are stored in the optical data code.

In an embodiment of the present invention, the optical data code is a linear data code such as a bar code, or a two-dimensional data code such as a matrix code, including a QR code and
/or data matrix code. Other matrix codes such as Aztec Code or PDF417 Code may also be used.

In an embodiment of the present invention, the size of the optical data code is larger than 10 mm², preferably larger than 30 mm². In an embodiment of the present invention, the size of the optical data code is smaller than 900 mm², preferably smaller than 400 mm², further preferably smaller than 200 mm².

In an embodiment of the present invention, the number of data elements of the optical data code is larger than 20, preferably larger than 35, preferably larger than 50, preferably larger than 100, further preferably larger than 200. In an embodiment of the present invention, the number of data elements of the optical data code is smaller than 10000, preferably smaller than 2000.

In an embodiment of the present invention, the optical data code has a storage capacity of at least 10 alphanumeric characters, preferably of at least 20 alphanumeric characters, more preferably at least 35 alphanumeric characters. In an embodiment of the present invention, the optical data code has a storage capacity of less than 5000 alphanumeric characters, preferably of less than 500 alphanumeric characters.

The present invention further comprises a medical peristaltic pump comprising a stator with a pump bed formed therein for receiving a pump hose segment inserted into the pump bed in arcuate form, a rotor for acting on the pump hose segment, a receiving area for receiving a connector connecting the pump hose segment to the pump bed, and an optical sensor for reading an optical data code, in particular a barcode, OR-code or a data matrix code, wherein the optical sensor is configured to read an optical data code arranged on the connector.

The medical peristaltic pump has the same advantages as already discussed above with respect to the inventive connector.

Further, it may be configured to receive a connector and/or pump hose set as described above and in the following. Further, the medical peristaltic pump may be configured as already discussed above in the context of the inventive connector and pump hose set as well as discussed in the following.

According to an embodiment of the present invention, the optical sensor and/or the lighting unit is a hand-held device.

According to this embodiment, a user of the pump will manually guide the optical sensor provided in a hand-held device to the connector to scan the optical data code. Preferably, the optical data code is provided on an upper surface of the connector, such that it can be read by the hand-held device while the connector is already connected to the pump.

According to an embodiment of the present invention, the optical sensor and/or the lighting unit is provided with a distance to the receiving area of the pump, such as on a side surface of a housing of the pump.

According to this embodiment, a user of the pump will manually guide the connector to the sensing area of the sensor and/or the lighting unit before connecting it to the pump.

However, according to a preferred embodiment of the present invention, the optical sensor and/or the lighting unit is arranged in the receiving area of the pump. Thereby, the optical data code can be automatically scanned by the optical sensor once the connector is arranged in the receiving area of the pump.

According to an embodiment of the present invention, the optical sensor and/or the lighting unit is arranged in a side surface of the receiving area, and is in particular configured to read an optical data code provided on a side surface of the connector.

According to a preferred embodiment of the present invention, the optical sensor and/or lighting unit is however arranged in a bottom surface of the receiving area. In particular, the optical sensor is arranged and configured to read an optical data code arranged on a bottom side of a connector received in the receiving area, with the bottom side of the connector facing the bottom side of the receiving area.

According to an embodiment of the present invention, the optical sensor has an optical axis that is parallel to an axis of the rotor.

According to a preferred embodiment of the present invention, the optical sensor however has an optical axis that is at an angle that is not zero with an axis of the rotor and/or the optical sensor has an optical axis that is not perpendicular with a surface of the optical data code. The inventors have found that due to reflections on the optical data code and/or a window of the optical sensor, it is favourable to look in an angle through the window and/or to the code.

In an embodiment, the optical sensor has an optical axis that is at an angle with an axis of the rotor and/or a direction normal to the surface of the optical data code that is larger than 10° and/or smaller than 50°.

According to an embodiment of the present invention, the medical peristaltic pump further comprises an ejector for ejecting the connector, the ejector being arranged in the receiving area. The ejector may in particular comprise an ejector pin that is moved by an actuator of the pump to eject the connector.

According to an embodiment of the present invention, the optical sensor and/or the lighting unit and/or a window for the optical sensor and/or the lighting unit and the ejector are arranged with a distance to each other in a bottom surface of the receiving area.

According to an embodiment of the present invention, the medical peristaltic pump comprises a lighting unit for lighting the optical data code when the connector is connected to the medical peristaltic pump for reading the optical data code.

According to an embodiment of the present invention, the lighting unit emits colored light.

According to an embodiment of the present invention, the lighting unit emits colored light having a complementary color with respect to a base color of the connector.

The present invention further comprises a medical device, in particular a blood treatment machine, in particular a dialysis machine, comprising a medical peristaltic pump as described above and in the following.

According to an embodiment of the present invention, the medical device comprises a controller programmed to adjust an operation of the medical device based on data read by the optical sensor from the optical data code.

The controller may comprise a microcontroller and a computer program stored in a non-volatile memory of the controller, wherein the computer program comprises instructions that will, when the computer program runs on the microcontroller, cause the medical device to operate as described above or in the following.

The controller is in particular connected by a data connection, such as a data bus, to the optical sensor for receiving data from the sensor when the sensor is reading the optical code, in particular image data. The controller may further be connected to an input and/or output device, in particular to a display of the medical device, and/or to actuators of the medical device, such as a drive of the pump, valve actuators for controlling fluid flow, etc. The controller may further be connected to other sensors of the medical device, such as a pressure sensor.

According to an embodiment of the present invention, the data may be used for different functionalities of the medical device.

According to an embodiment of the present invention, the controller is programmed to use the data read from the optical data code for at least one out of or any combination of:
- documentation
- in case of a code not recognized by the controller, providing indication to user that an unknown pump hose set, and therefore possibly a pump hose set that is not compatible with the medical device, and/or a pump hose set that is not non-verified/validated for the device to which the hose set should be connected, is intended to be used. Possibly, such an indication may include an indication that the liability that no patient risk is associated with this pump hose set is with the user. Such an indication may be provided to as information on a screen of the medial device connected to the controller.
- performing an emptying and/or priming process, in particular an adapted and/or optimized emptying and/or priming process. Preferably, the emptying and/or priming process may be performed based on information regarding a volume and/or behavior of the pump hose set derived from the data read from the optical data code. Performing the emptying and/or priming process may include performing the process with a rinsing volume and/or emptying time based on the data provided on the optical data code. The emptying and/or priming process may comprise that the controller controls the action of the pump.
- Activating features of the device based on the data. For example, data information read from the optical data code may be necessary to perform certain features for patient safety of the feature. If no code is present or the relevant data are absent, then such a feature will not be activated. Further, depending on the type of connector, the controller may enable a further sensor such as a pressure sensor, knowing from the code that an optional component collaborating with the sensor is part of the bloodline.
- Blocking execution of one or more feature(s) such as an automatic priming and/or automatic emptying, on general or based on the data provided on the optical data code.

The present invention further comprises a set comprising the inventive medical peristaltic pump and/or the medical device and an inventive connector and/or an inventive pump hose set.

The present invention further comprises a method for manufacturing a pump hose set as described above and in the following, comprising the steps of:
- manufacturing the connector,
- connecting the pump hose segment to the connector and preferably connecting the connecting hose segments to the connector and
- sterilizing the tubing set,
wherein a optical data code is provided on the connector.

According to an embodiment of the present invention, the optical data code is provided on the connector before the step of sterilizing.

According to an embodiment of the present invention, the optical data code is provided on the connector after connecting the pump hose segment and/or the connecting hose segments to the connector.

Thereby, it is possible to store data indicative of the specific pump hose set in the optical data code. Further, errors in associating data specific to the pump hose segment and/or connecting hose segments connected to the connector to the optical data code can be more easily avoided. Further, it is avoided that the optical data code is impaired or destroyed when the pump hose segment and/or connecting hose segments are connected to the connector, e.g. by a glue used for the connection.

In an alternative embodiment, however, the optical data code is provided on the connector before connecting the pump hose segment and/or the connecting hose segments to the connector.

Preferably, the optical data code provided on the connector is covered during the step of connecting the pump hose segment and/or the connecting hose segments to the connector, in particular by a detachable cover and/or by gripping elements comprising a cover. Thereby, the optical data code is protected by the cover while the pump hose segment and/or connecting hose segments are connected to the connector.

Further, a cover placed on the optical data code may also be used with any one of the embodiments described herein, in order to protect the optical data code. In an embodiment, the cover is transparent, and can therefore remain on the connector.

In an embodiment, that may be combined with any one of the embodiments described so far, the connector therefore comprises a transparent cover placed on top of the optical data code. The transparent cover may in particular be a transparent plastic film.

The present invention will now be described in more detail on the basis of embodiments and drawings.

The drawings show:
- Fig. 1: a first embodiment of an inventive connector and an inventive pump hose set comprising the connector;
- Fig. 2: an embodiment of an inventive medical peristaltic pump, with an embodiment of an inventive pump hose set inserted into the pump bed of the medical peristaltic pump,
- Fig.3: the pump bed of the embodiment of the medical peristaltic pump as shown in Fig. 2,
- Fig. 4a and 4b: top view and sectional view of the embodiment of the connector shown in fig. 1,
- Fig. 5a to 5c: perspective views of four variants of a second embodiment of an inventive connector,
- Fig. 6a and 6b: views of two variants of a third embodiment of an inventive connector included in an embodiment of a pump hose set,
- Fig. 7: a top view of a fourth embodiment of an inventive connector included in an embodiment of a pump hose set,
- Fig. 8: a top view of a fifth embodiment of an inventive connector included in an embodiment of a pump hose set,
- Fig. 9: two schematic diagrams showing the laser writing of the optical data code on flat surface and on curved surface.

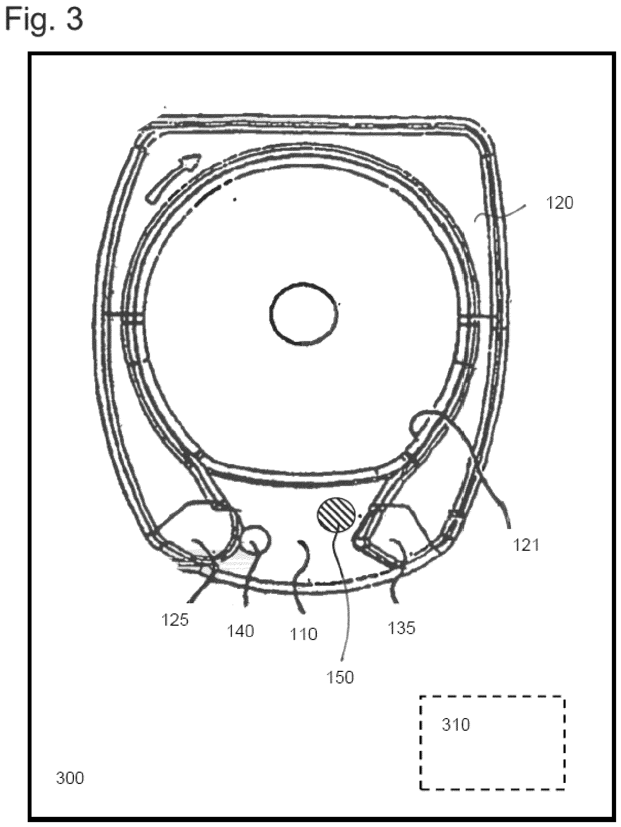

The general configuration of all the embodiments of the present invention of a connector 10 for positioning a pump hose segment 1 in a pump bed of a medical peristaltic pump 10 in arcuate form will now be described. The invention could however also be applied to embodiments where the pump hose segment extends linearly in the pump bed, such as a finger pump. The medical peristaltic pump 10 is part of a medical device 300, such as a dialysis machine, schematically shown in Fig. 3. The medical device 300 may comprise a controller 310 as described above.

In the embodiments, the connector 10 comprises a first fluid path 20 associated with the first end 5 of the pump hose segment and a second fluid path 30 associated with the second end 4 of the pump hose segment.

In particular, the first end 5 of the pump hose segment 1 is connected to the first fluid path 20 and the second end 4 of the pump hose segment 1 is connected to the second fluid path 30. Further, a first connecting hose segment 2 is connected to the other end of the first fluid path 20 of the connector, and a second connecting hose segment 3 is connected to the other end of the second fluid path 30 of the connector.

For this purpose, the connector 10 comprises a first pump hose receiving section 21 associated with the first end 5 of the pump hose 1 and a second pump hose receiving section 31 associated with the second end of the pump hose 1. Further, the connector 10 comprises a first connecting hose receiving section 22 associated with the first connecting hose segment 2 and a second connecting hose receiving section 32 associated with the second connecting hose segment 3.

The hose segments are fluidly connected to the associated receiving sections of the connector, e.g. by being inserted into the receiving sections and/or glued to the receiving sections.

The first fluid path 20 connects the first connecting hose receiving section 22 with the first pump hose receiving section 21 and the second fluid path 30 connects the second connecting hose receiving section 32 with the second pump hose receiving section 31.

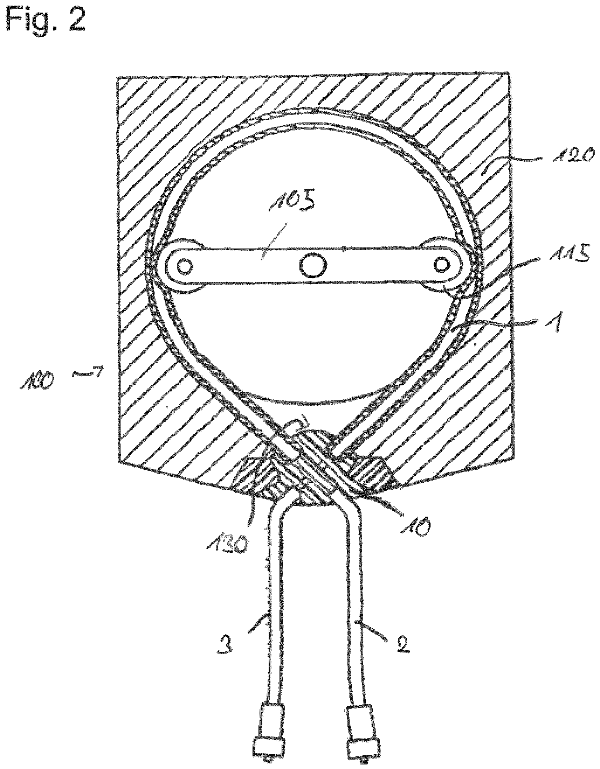

An embodiment of the medical peristaltic pump 100 with the pump hose segment 1 and the connector 10 inserted into the pump bed 121 is shown in Fig. 2 and 3.

The medical peristaltic pump 100 comprises a stator 120 including an arcuate pump bed 121 into which the pump hose segment 1 is inserted in the form of a pump loop, and a rotor 105 rotating with in the pump bed 121 and comprising at least two pressing elements 115 pressing against the pump hose segment 1 such that the pump hose segment is compressed between the pressing elements 115 and the pump bed. Thereby, a section of the pump hose 1 between two adjacent pressing elements 115 is closed off from the remainder of the pump hose segment. Thereby, on rotation of the rotor 105, the fluid within the pump hose segment is pumped from the first end 5 to the second end 4 of the pump hose segment or vice versa. The pressing elements 115 may in particular be rollers, such that the pump is configured as a roller pump.

According to the present invention, an optical data code 50 is provided on the connector 10. Further, the medical device or the medical peristaltic pump is provided with an optical sensor for reading the optical data code 50 of the connector 10 when the connector 10 is connected to the medical peristaltic pump.

In particular, the pump and/or the medical device may have a sensor window 150 provided in its housing, with an optical sensor for reading the optical data code arranged behind the sensor window 150 inside the pump and/or inside the medical device.

The optical sensor may in particular be a camera, such as a 2D CCD camera, a CMOS Camera, a 3D Camera, and/or an infrared camera.

Placing the optical data code on the connector 10 has several significant advantages:
First of all, the connector is very well defined (actually better defined than most other components) in its position and firmly fixed. This allows for a reliable interface definition to the optical sensor integrated in the pump for reading the optical data code and there are little tolerances with respect to the misalignment of the optical data code and the optical sensor. In particular, the connector will not move relative to the sensor even when forces act on the pump hose segment, such as during insertion or when the pressing elements 115 of the rotor are pressed against the pump hose segment. In contrast to placing the data code on the pump hose segment, placing the data code on the connector will therefore ensure that it will not move relative to the sensor.

Further, the medical peristaltic pump and, therefore, the connector for the pump hose segment is present in every tubing set also in case different variants of the tubing sets may be used with the medical device comprising the medical peristaltic pump. Further, the medical peristaltic pump is a complex and crucial building block which may be used in different variants of the medical device and also remains often unchanged even if the medical device is further developed. Thus, the interface between connector and sensor remains regularly unchanged and is not to be modified/adapted in case the device is continued to be developed. Finally, in a usual medical device, the area of the medical peristaltic pump is an area that needs to be cleaned carefully anyway after every treatment. Accordingly, the machine side of the interface between sensor and connector is automatically subject to regular cleaning and, accordingly, a deterioration of the optical system is of low risk as the sensor window 150 for the optical sensor is also regularly cleaned.

The medical device may in particular be a dialysis machine.

The dialysis machine may have a housing, wherein the pump is positioned on one side of the housing, in particular on a front plane of the housing.

The pump may in particular be a blood pump. The dialysis machine may include further pumps, such as a dialysate and/or substituate pump and/or a heparin pump.

The connector may be part of a blood tubing set connectable to the dialysis machine, the blood tubing set comprising a venous and an arterial line and/or a dialyzer, such as a hollow membrane dialyzer.

Embodiments of the optical data code, the connector and the placement of the optical data code on the connector are described in the following.

The optical data code 50 may be a linear data code such as a bar code, or a two-dimensional data code such as a matrix code, including a QR code or a data matrix code.

In the embodiment, the size of the optical data code is 8x8 mm and the number of data elements is 26x26, but different values are possible depending on the amount of data required and the space available. For example, a larger number of data elements may be required if the data code includes a key for data encryption.

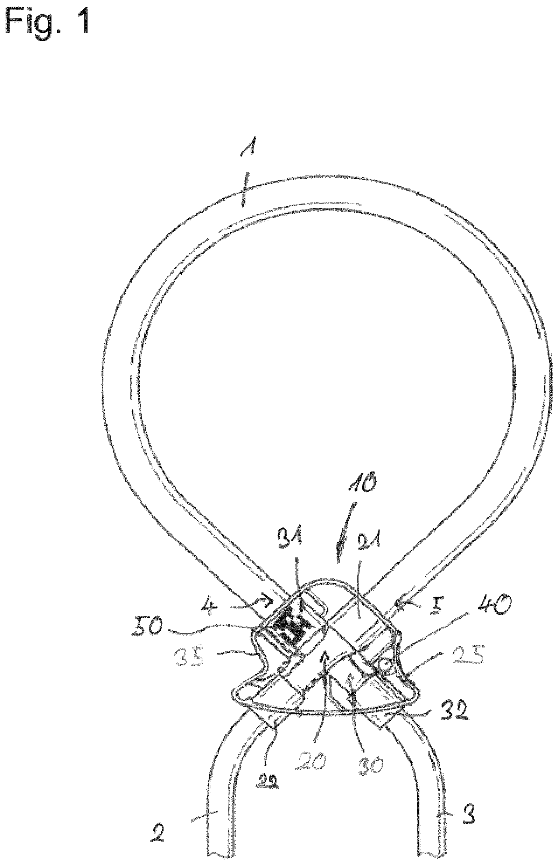

In some of the embodiments, such as shown in Fig. 1, 4, 5a and 5b, 6 and 7, the optical data code 50 is positioned on the first or second pump hose receiving section 21 or 31, and in particular on the second pump hose receiving section 31.

The outer diameter of the pump hose receiving sections 21 and 31 is bigger than the outer diameter of the connecting hose receiving sections 22 and 32. Therefore, there is more space available for placing the optical data code.

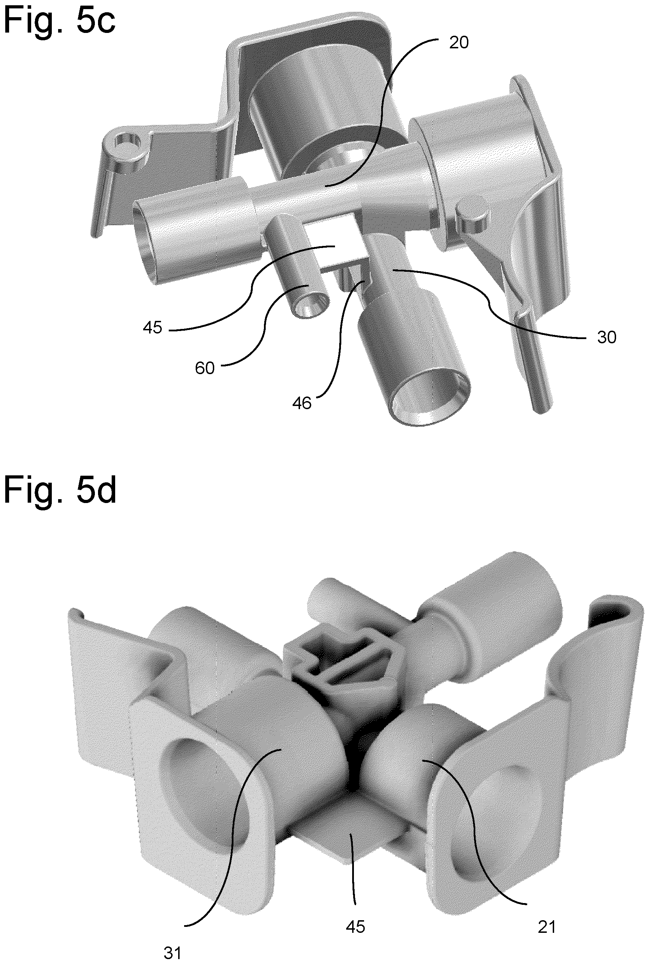

In the embodiments shown in Fig. 5c and 5d, in contrast, a plate element 45 is provided on which the optical data code is provided. In the embodiments, the plate element extends in a plane that is parallel to a plane defined by the axes of the two pump hose receiving sections 21 and 31 and/or at a right angle to the axis of the rotor of the pump. In particular, the plate element has a flat surface on which the optical data code is provided.

In these embodiments, the plate element 45 is attached to at least one of the.tubular element defining the first fluid path 20, the second fluid path 30 and/or the further hose receiving section 60.

In particular, it extends between at least two of the tubular elements and is connected to both, therefore increasing the stability of the connection.

In the embodiment shown in Fig. 5c, the plate element 45 is provided between the tubular elements of the second fluid path 30 and the further hose receiving section 60. Further, element 46 connects the plate element 45 also to the tubular element of the first fluid path 20. The plate element 45 is provided adjacent to the position where the first fluid path 20 and the second fluid path 30 cross each other.

In the embodiment shown in Fig. 5d, the plate element 45 is provided between the tubular elements forming the pump hose receiving sections 21 and 31.

In the embodiment, the optical data code 50 is positioned on the bottom side of the connector facing the pump bed. This bottom side of the connector 10 is shown in Fig. 1. Positioning the optical data code 50 on the bottom side will avoid stray light from deteriorating the reading of the code. Further, it makes the placement of the optical sensor and/or the window 150 for the optical sensor on the pump easier.

In particular, the pump 100 comprises a connector receiving area 110, arranged in the embodiment in a pump bed opening between the ends 125 and 135 of the pump bed 121, for receiving the connector 10. The pump bed 121 and the pump bed opening 110 comprises a bottom wall extending in a plane that is perpendicular to the axis of rotation of the rotor 105. The window 150 for the sensor is positioned in this bottom wall of the pump bed opening / connector receiving area 110. Preferably, an optical axis of the sensor extends at an angle that is not zero with respect to the axis of rotation of the rotor 105 and/or a surface of the window 150. This reduces reflections and improves reading of the optical data code.

The optical data code could be applied to the connector by printing with ink or etching. Further, the optical data code can be provided on a sticker attached to the connector.

In a preferred embodiment, however, laserwriting is used for applying the optical data code to the connector. The main advantages are that laserwriting is simple, efficient, made without any sort of ink and content can be easily modified without any special setup. Therefore, there is no need for any kind of consumable or spare parts for laser writing. Further, it is potentially less toxic (no ink is used) and is also insensible against sterilization.

In the embodiments, the connector is made from a base material having a base color. The base color is uniform at least in the area where the optical data code is applied.

Because the color of the optical data code depends on base color and the number of passes, an appropriate base color and a sufficient number of passes is required to achieve a sufficient contrast.

In one embodiment, the base color is red, and the laser writing is white. Alternatively, the base color is white and the laser writing is black. Further alternatively, the base color is blue and the laser writing is preferably white.

In a further embodiment, the base color of the connector may be transparent. In particular, if the connector is a cassette, the base color of the element of the cassette on which the optical data code is provided may be transparent.

Additionally, the contrast is not only influenced by the connector and the optical data code itself, but also by the lightning used from the machine side.

In an embodiment, the optical sensor of the medical peristaltic pump of the present invention comprises a lighting unit for lighting the optical data code during reading. In an embodiment, the lighting unit emits colored light. In particular, the lighting unit emits colored light of a complementary color to the base color of the connector to reach a better contrast.

In the embodiments, the optical data code is positioned on a non-flat surface of the connector and in particular on the rounded exterior surface of the pump hose receiving section.

In alternative embodiments, the code is applied to a flat surface of the connector.

For example, if the connector is a cassette, the optical data code is preferably provided on a flat surface of the cassette, in particular on a flat part of the lower or upper surface of the cassette.

Further, also for connectors that do not have the form of a cassette, there may be flat surfaces on which the optical code may be provided. Preferably, such a surface extends on a bottom side of the connector, for example between the pump hose receiving sections. The flat surface may in particular be provided on a plate-like element of the connector.

In the embodiments, the optical sensor may be provided by a digital camera. The optical sensor may comprise a lense system for compensating for a non-flat focus reading region.

Further, the 3D-shape of a non-flat surface of the connector where the optical data code is placed, and/or due to the optical distortion of the optical sensor, which may occur due to the wide angle camera necessary to realize a very short distance between camera and code, may lead to a distortion of the optical data code in the image of the optical sensor.

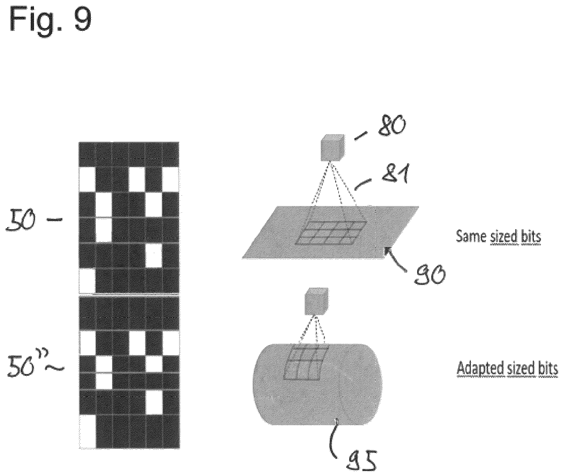

In an embodiment, such a distortion can be compensated by an opposite distortion of the optical data code that is provided on the connector. As can be seen from Fig. 9, the rounded shape of the non-flat surface 95 leads to a distortion of the data code in the image of the sensor, which is compensated by applying the optical data code 50" in an oppositely distorted manner on the non-flat surface as compared to the non-distorted data code 50 on the flat surface 90. In principle, a distortion due to the camera optics may be compensated in the same manner.

As shown in Fig. 9, any shape can be laser printed on any surface. Therefore it is possible to print a distorted code, if the picture of the code is generated in distorted form and then sent to the laser marker for laser marking.

In an embodiment of a method for manufacturing the connector and/or the pump hose set, the optical data code is applied to the connector before sterilization but after assembly of pump hose set, i.e. after the pump hose segment 1 and the connecting hose segments 2, 3 have been connected to the connector 10.

After the application of the optical data code, the pump hose set is packaged and/or sterilized.

In an alternative solution, the optical data code may be applied to the connector before it is connected to the pump hose segment and/or after it is connected to the pump hose segment but before it is connected to the connecting hose segments.

The inventors have found out that there may be problems in the manufacturing of the connector if the code is attached before the tubes. In particular, solvent for attaching the tubes to the connector can come through the fingers of the factory workers or in the form of drops on the already attached code, which can result in damage to the code, making it difficult or impossible to read.

Therefore, providing the code to the connector after attaching the tube segments to the connector will avoid these problems.

An alternative production method is that the code is already provided on the connector before the hoses are fitted, whereby the code is covered when the hoses are subsequently fitted, for example by a cover that preferably has the shape of the connector. The cover can, for example, be clicked onto the connector or attached in some other way.

Another option would be to use gripping pliers or pliers with a cover that holds the connector and at the same time covers the code.

Since the connector including the tubing set is only sterilized after all components have been assembled, a reusable cover could be used.

One embodiment can also provide for the cover (e.g. in the form of a film) to be permanently attached to the code. In this case, the cover is made of transparent material, such that the connector can be used with the cover attached to the connector. Such a cover may be used with any of the embodiments of a connector described herein.

The elements of the connector discussed so far are present in all the embodiments of the present invention, where not explicitly indicated in the following.

In the following, further features will be discussed, which are not necessarily present in all embodiments.

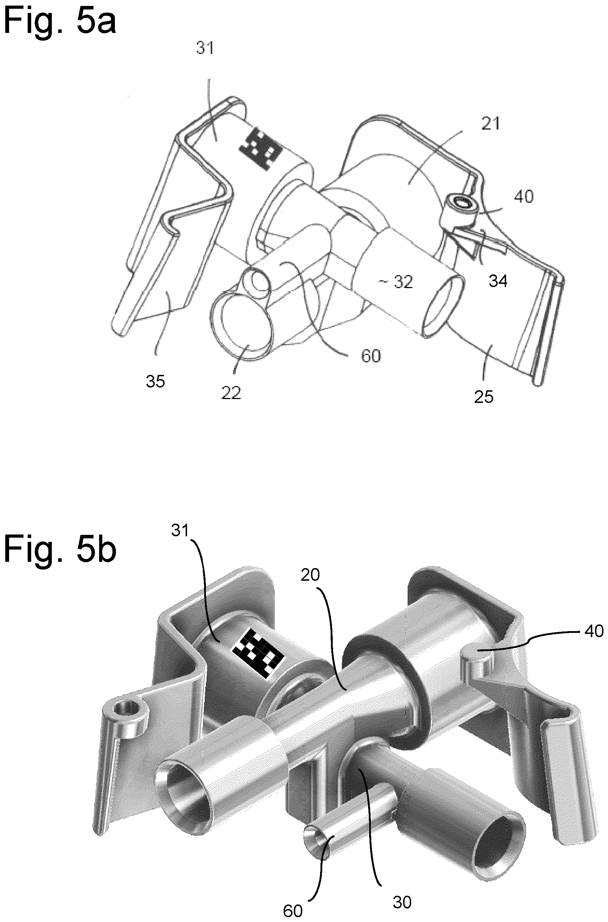

The following features are present in some embodiments, and in particular the embodiment shown in Fig. 1 to 4, as well as the embodiment of Fig. 5.

In these embodiments, the pump 100 comprises an ejector 140 for ejecting the connector from the pump bed opening / connector receiving area 110. In particular, the ejector comprises an ejector pin actuated by an actor, which is urged against the connector 10 to dislocate the connector from the pump bed opening / connector receiving area 110.

As can be seen in Fig. 1 and Fig. 5, the connector 10 comprises, on its bottom side, an abutment element 40 engaging with the ejector pin of the ejector 140. The side of the connector 10 comprising the abutment element 40 is therefore the bottom side.

In the embodiment, the optical data code 50 is therefore arranged on the same side as the abutment element 40 to which the ejector 140 of the pump 100 engages. The abutment element 40 may in particular have a ring shape, with the ejector pin engaging with the center of the ring shape.

As can be seen from Fig. 3, the ejector 140 and the optical sensor are arranged with a distance to each other in the pump bed opening / connector receiving area 110. In particular, a window 150 for the sensor may be provided in the housing of the pump in the pump bed opening / connector receiving area 110 at a distance to the ejector 140.

In the embodiment, the connector 10 is at least in part arranged between the ends 125 and 135 of the pump bed when connected to the pump.

The following features are equally present in some embodiments, and in particular the embodiment shown in Fig. 1 to 4, as well as the embodiment of Fig. 5.

In these embodiments, the connector 10 is configured to be clipped between the ends 125 and 135 of the pump bed.

For this purpose, as can be seen in Fig. 1 to 5, the connector 10 comprises clip elements 25 and 35 cooperating with mating surfaces of the ends 125 and 135 of the pump bed to provide a clip connection. In particular, the clip elements 25 and 35 are flexible, resilient elements than are deformed during insertion into the pump bed opening 110 and held by mating surfaces of the ends 125 and 135 of the pump bed. The mating surfaces of the ends 125 and 135 of the pump bed therefore constitute counter elements of the clip connection.

In the embodiment, the clip elements 25 and 35 have different shapes in order to provide a keyed clip connection. In particular, the first clip element 25 has a rounded shape and the second clip element 35 has an angled shape, corresponding to the rounded shape of the mating surfaces of ends 125 and 135 of the pump bed.

The ejector 140 is provided on the side of the first, rounded end 125 and therefore the abutment element 40 is provided on the side of the first, rounded clip element 25, because this allows for an easier ejection of the connector. In particular, the clip arm 25 comprises a protrusion 34 on which the abutment element 40 is located.

Further, the first, rounded clip element 25 is provided, in the embodiment, on the side of the first pump hose receiving section 21 and in fact in the quadrant of the connector between the first pump hose receiving section 21 and the second connecting hose receiving section 32.

Consequently, the angled clip element 35 is provided, in the embodiment, on the side of the second pump hose receiving section 31 carrying the optical data code 50. Further, the optical sensor and/or the window 150 for the optical sensor is provided on the side of the angled end 135 of the pump bed.

In the embodiment, the minimum distance between the center line of the ejector 140 and the optical sensor 150 is larger than the minimum distance between the center line of the ejector 140 and the end 125 of the pump bed opening that is closer to the ejector 140 and larger than the distance between the center line of the optical sensor and the end 135 of the pump bed opening that is closer to the optical sensor, and in particular larger by a factor of at least 1,5.

The following features are present in some embodiments, and in particular the embodiments shown in Fig. 1 to 4, 5, 6 and 8.

In these embodiments, the first and second fluid path 20 and 30 are at least partly contained within a common connector part, the common connector part in particular comprising the first and second pump hose receiving section. The common connector part may be constructed as a single component, and in particular formed integrally as one piece, in particular by injection molding.

In the embodiments shown in Fig. 1 to 5, the clip elements 25 and 35 are integrally formed as part of the common connector part, for example by injection molding. In an alternative embodiment not shown, the clip elements 25 and 35 may be provided on a separate part of the connector, that is connected to the common connector part comprising the first and second pump hose receiving section and/or the first and second fluid path, e.g. by gluing, welding or a form-fit or clip-connection.

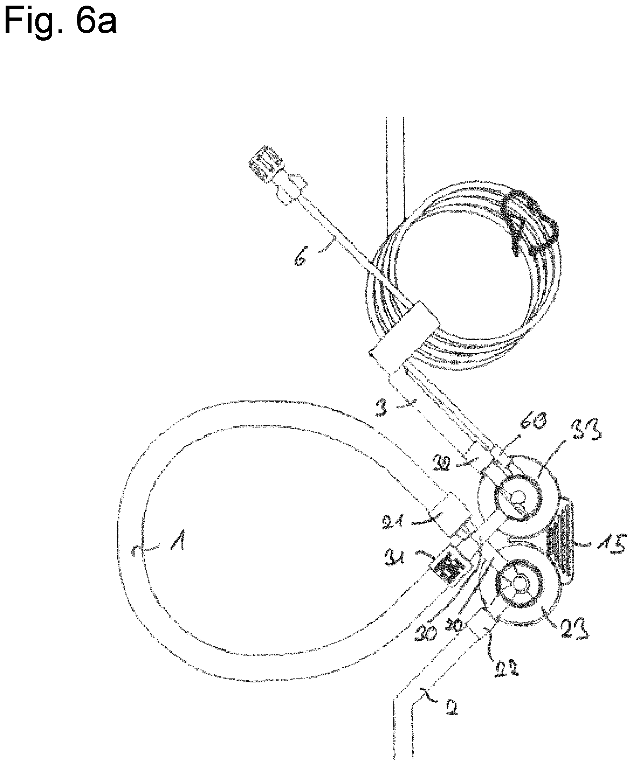

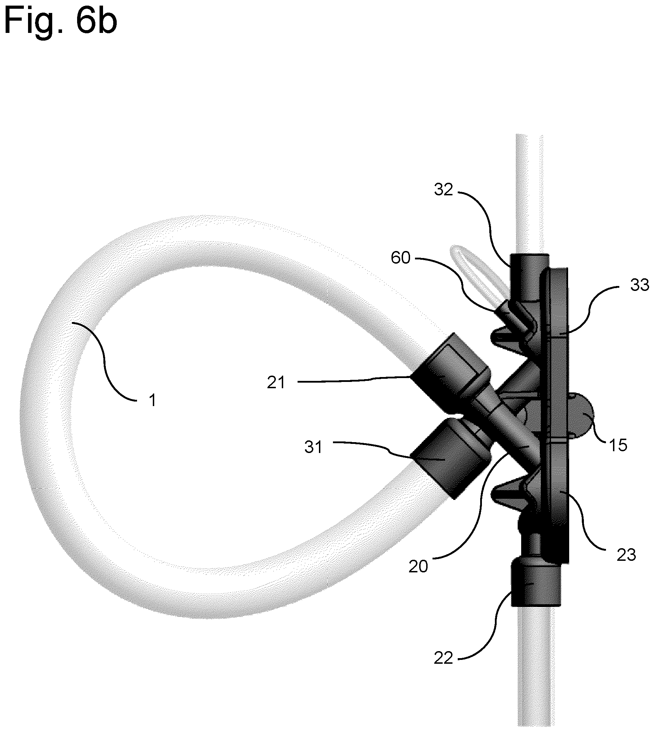

In the embodiment shown in Fig. 6a, and 6b, the elements for connecting the connector to the pump are also integrally formed as part of the common connector part, for example by injection molding.

In a further aspect, in these embodiments, the first and second fluid path 20 and 30 cross within the connector. In particular, the first and second fluid path 20 and 30 cross within the common connector part. In particular, the first and second fluid path 20 and 30 cross in the area between the ends 125 and 135 of the pump bed, such that the fluid streams will cross where they exit and enter the pump bed, respectively.

The first fluid path 20 may cross the second fluid path 30 on the side facing away from the pump bed, and in particular on the opposite side on which the abutment element 40 is provided, as shown in Fig. 5a. Alternatively, as shown in Fig. 5b, the first fluid path 20 may cross the second fluid path 30 on the side facing towards the pump bed, and in particular on the same side on which the abutment element 40 is provided.

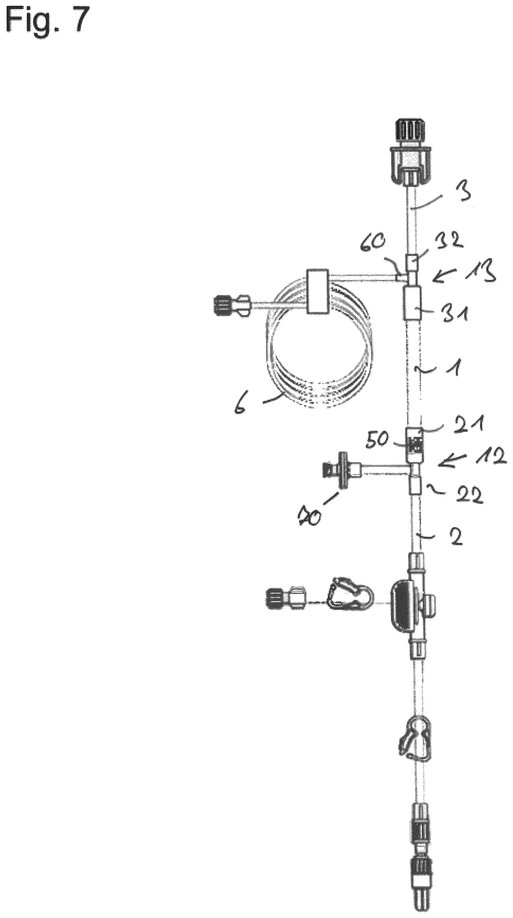

In contrast, in the embodiment shown in Fig. 7, the connector comprises separate connector parts 12 and 13 comprising the first and second fluid path, respectively. In particular, the first connector part 12 comprises the first pump hose receiving section 21 and the first connecting hose receiving section 22, and the second connector part 13 comprises the second pump hose receiving section 31 and the second connecting hose receiving section 32.

Each of the connector parts 12 and 13 may be formed integrally, e.g. by injection molding.

Further, as can be seen from Fig. 7, the first connector part 12 comprises a further hose receiving section for connecting a hose leading to an interface 70 for connecting the pump hose set to a pressure transducer interface. The interface 70 may comprise a hydrophobic membrane.

The following features are present in some embodiments, and in particular the embodiment shown in Fig. 5, 6 and 7.

In these embodiments, the connector comprises a further hose receiving section 60 for receiving a further hose 6. The hose 6 may be used for heparin infusion.

The hose receiving section 60 is fluidly connected to the second fluid path 30 of the connector in the embodiments, but may equally be connected to the first fluid path.

The hose receiving section 60 may be provided by a tubular element.

In Fig. 5a, this tubular element extends parallel to the first fluid path 20 and is provided directly on the tubular element forming the first fluid path 20. In the alternative embodiment shown in Fig. 5b, the tubular element of the hose receiving section 60 extends at a distance to the tubular element forming the first fluid path 20.

In Fig. 6a and 6b, the tubular element of the hose receiving section 60 is connected to the pressure measurement cavity 33 described later and extend parallel to the tubular element forming the second connecting hose receiving section 32.

In Fig. 7, the tubular element of the hose receiving section 60 is part of the second connector part 13 and is attached to the tubular element forming the second fluid path at right angles.

The following features are present in some embodiments, and in particular the embodiment shown in Fig. 1 to 4, 5 and 7.

In these embodiments, the first fluid path 20 and the second fluid path 30 are each formed by tubular elements comprising the respective pump hose receiving section and connecting hose receiving section. The tubular elements may have a varying outer and/or inner diameter.

In the embodiments, the pump hose receiving section and the connecting hose receiving section of the first fluid path 20 are extending in the same direction and are in particular parallel and more particularly coaxial. Further, the pump hose receiving section and the connecting hose receiving section of the the second fluid path 30 are extending in the same direction and are in particular parallel and more particularly coaxial.

The following features are present in the embodiments shown in Fig. 1 to 4 and 5. In these embodiments, the entire connector 10 is formed integrally as a single part, in particular by injection molding.

In Fig. 1 to 4, the ends of the tubular elements forming the first and the second fluid path 20 and 30, which cross in the connector, are connected by a side wall extending around the connector, the side wall including the clip elements 25 and 35.

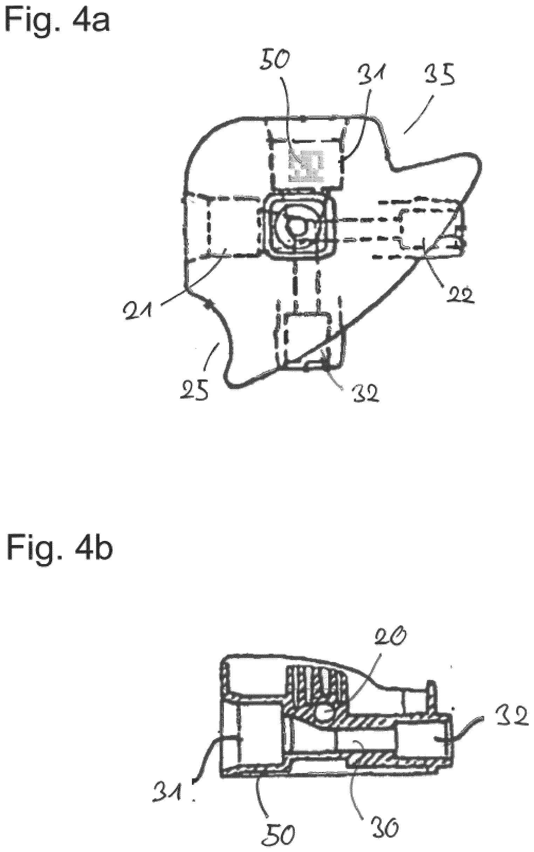

The tubular elements of the first and the second fluid path 20 and 30 are connected where they cross each other, see Fig. 4.

Further, the tubular elements of the first and the second fluid path 20 and 30 may be connected to each other and the side wall also in areas of the connector.

In Fig. 5a and 5b, in contrast, the tubular elements of the first and the second fluid path 20 and 30 are only connected where they cross each other. In Fig. 5c and 5d, in addition, they are connected by a plate element 45 carrying the connector and extending in parallel to a plane defined by the axes of the fluid paths 20 and 30. In all the embodiment of Fig. 5a to 5d, the connector does not comprise a side wall connecting the ends of the tubular elements of the first and the second fluid path 20 and 30 with each other.

In these embodiments, the clip elements 25 and 35 are formed by clip arms. The clip arms 25 and 35 respectively extend from the outer end of the tubular elements of the first and the second fluid path 20 and 30 at the side of the pump hose receiving sections 21 and 31 and have a free end.

The clip arms have the shape of a plate extending in a plane that is perpendicular to a main plane of the connector defined by the direction of the fluid paths, and are bent to provide the rounded and angled shape, respectively.

In the embodiment, the abutment element 40 is provided on one of the clip arms, and in the embodiment on clip arm 35 connected to the pump hose receiving section 21.

In an alternative embodiment to the one shown in Fig. 5a to 5d, only one of the clip arms 25 and 35 has a free end, while the other clip arm is part of a side wall that connects the respective pump hose receiving section with one of the connecting hose receiving sections, as in the embodiment shown in Fig. 1.

In a further alternative embodiment, the clip arms and/or the side wall may be part of a first part of the connector, and the first and the second fluid path including the pump hose receiving sections and the connecting hose receiving sections may be integrated in a second part of the connector, which are attached to each other, e.g. by gluing, welding or a form-fit- or clip-connection.

In some of the embodiments, such as those shown in Fig. 1, 4, 5a, 5b, 6 and 7, the optical code 50 is provided on one of the pump hose receiving sections 21, 31, and in particular on the second pump hose receiving section 31.

In particular, in the embodiments shown in Fig. 5a and 5b, the optical data code is provided on the second pump hose receiving section 31 to which the clip arm 35 that does not carry the abutment element 40 is connected.

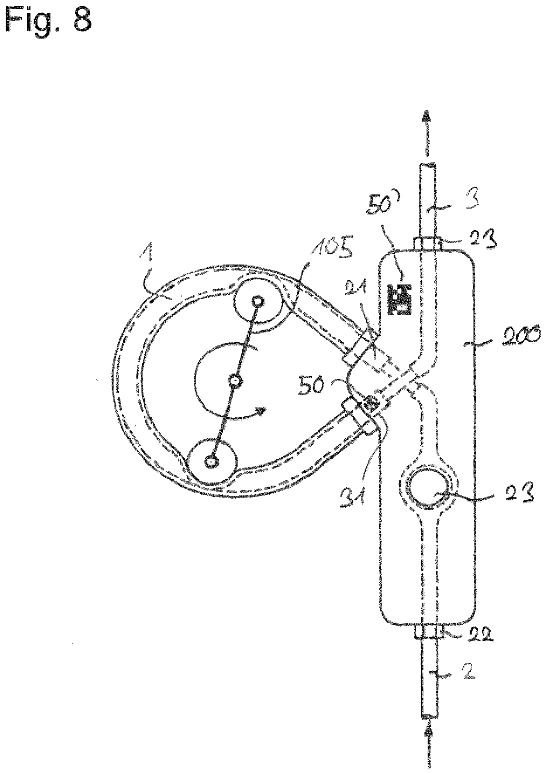

The following features are present in some embodiments, and in particular the embodiment shown in Fig. 6 and 8.

In these embodiments, at least one functional element is integrated in the connector. The functional element may in particular include one or more out of a pressure transducer interface, an air trap, in particular a venous air trap, a venous clot catcher, a substitute connection port, a single-needle chamber, a heparin line with non-return valve, a rinsing plug.

In some of the embodiments, at least one pressure transducer interface for connecting to a pressure transducer is integrated in the connector. In the embodiments, the pressure transducer interface comprises a flexible membrane covering a cavity within the connector. The flexible membrane of the pressure transducer interface can be coupled to a pressure transducer to measure the pressure inside the cavity. The cavity is connected to the first or the second fluid path in order to measure the pressure downstream or upstream of the pump hose.

In the embodiments shown in Fig. 6a and 6b, a pressure transducer interfaces 23 and 33 is provided for each of the fluid paths 20 and 30. In the embodiment, the fluid paths each comprise a tubular element connecting the respective pump hose receiving section 21 and 31 with a cavity of the pressure transducer interface 23 and 33, with these tubular elements crossing each other. Further, tubular elements comprising the connecting hose receiving sections 22 and 32 branch off from the cavities of the pressure transducer interfaces 23 and 331. Further, a handle 15 is provided on the connector.

In the embodiment of Fig. 6a, the membranes of the pressure transducer interfaces 23 and 33 extend in a plane perpendicular to the axis of rotation of the pump and/or in a plane that is parallel to the plane defined by the pump hose segment 1 and/or defined by the axes of pump hose receiving sections 21 and 31. In the alternative embodiment shown in Fig. 6b, the membranes of the pressure transducer interfaces 23 and 33 extend in a plane than extends parallel to the axis of rotation of the pump and/or in a plane that extends at a right angle to the plane defined by the pump hose segment 1 and/or defined by the axes of pump hose receiving sections 21 and 31 .

In the embodiment shown in Fig. 8, the connector is provided as a cassette 200 including the pressure transducer interface 23. In this embodiment, the optical data code 50' may be provided on the body of the cassette, preferably on the bottom side of the body of the cassette. The cassette may include other or further functional elements such as an air trap, in particular a venous air trap, a venous clot catcher, a substitute connection port, a single-needle chamber, a heparin line with non-return valve, a rinsing plug.

Further, the cassette may be provided with two separate pump hose segments, connected by the cassette to two medical peristaltic pumps. In such an embodiment, the cassette may be provided with two pairs of first and second pump hose receiving sections, each one of the pairs connected to one of the pump hose segments.

The connectors of the present invention are preferably manufactured from plastic.

## Claims

1. A connector for positioning at least one pump hose segment in a pump bed of a medical peristaltic pump, in particular for positioning the at least one pump hose segment in the pump bed in arcuate form,
**characterized in that**
an optical data code, in particular a one-dimensional or two-dimensional barcode or a matrix code, in particular a QR Code or a data matrix code, is arranged on the connector.

2. The connector of claim 1, comprising a first fluid path associated with a first end of the pump hose segment and a first pump hose receiving section associated with the first fluid path for receiving the first end of the pump hose segment, and/or a second fluid path associated with a second end of the pump hose segment and a second pump hose receiving section associated with the second fluid path for receiving the second end of the pump hose segment,
the connector preferably comprising a first connecting hose receiving section associated with the first fluid path for receiving a first connecting hose segment and/or a second connecting hose receiving section associated with the second fluid path for receiving a second connecting hose segment.

3. The connector of claim 2, wherein the first fluid path and the second fluid path cross in the connector and/or wherein the connector comprises an integrally formed connector part comprising the first and the second fluid path, wherein preferably the connector and/or the connector part is formed as a single, integral piece, preferably by injection molding,
and/or
wherein the connector comprises a first connector part comprising the first fluid path and a second connector part comprising the second fluid path, wherein preferably each connector part is formed as a single, integral piece, preferably by injection molding.

4. The connector of one of claims 2 to 3, wherein the optical data code is arranged on a functional element of the connector, and in particular on an outer surface of an element of the first or second fluid path, and in particular on the first and/or second pump hose receiving section, wherein preferably, an inner and/or outer diameter of the first and/or second pump hose receiving section is larger than an inner and/or outer diameter of the first and/or second connecting hose receiving section.

5. The connector of one of claims 2 to 3, wherein the optical data code is arranged on an element specifically provided for placing the data code, such as a plate-like element extending from a functional element or an element protruding from a functional element towards the bottom side, and/or wherein the optical data code is arranged on an element having a flat surface.

6. The connector according to any of the preceding claims, wherein the connector is at least in part made of a formstable material, in particular of a plastic, such as polypropylene, and the data code is provided on the part made of the formstable material or the data code is provided on a part of the connector made of a non-formstable material with the part of the connector made of a non-formstable material being attached to the part made of the formstable material such that the position of code is fixed elative to the formstable material.

7. The connector according to claim 6, wherein the part made of the formstable material is connected to a connection part of the connector or comprises a connection part that is configured to be connected to a corresponding structure of the pump, wherein the position of the connector is fixed upon insertion into the corresponding structure of the pump, and in particular remains fixed during operation of the pump.

8. Connector according to the preceding claims, wherein during operation of the pump a movement, in particular any movement, of the code is smaller than a movement of a pump loop connected to the connector.

9. The connector of one of the preceding claims, wherein the optical data code is formed by laserwriting, wherein the optical data code is preferably written by at least two consecutive passes of laserwriting, and/or wherein the connector preferably has a base colour that is transparent, white, red or blue, and/or wherein the optical data code is formed on a non-flat surface of the connector, wherein preferably a distortion of the surface in an image of an optical sensor reading the optical data code is compensated by a distortion of the optical data code and/or wherein individual bits of the Code in a center area of the code are differently designed, in particular with a smaller area, than in an edge area of the code, in particular with a gradually increasing area from the center to the edge.

10. The connector of one of the preceding claims, wherein the optical data code is arranged on a bottom side of the connector facing a receiving area of the medical peristaltic pump,
wherein the connector preferably comprises at least one abutment element cooperating with at least one ejector of the medical peristaltic pump arranged on the bottom side of the connector for ejecting the connector,
wherein further preferably, in plan view, the connector has a first part comprising the first pump hose receiving section and a second part comprising the second pump hose receiving section, wherein the optical data code is arranged in one of the parts and the at least one abutment element in the other, remaining part, wherein the at least one abutment element is preferably arranged in an area of the connector extending between the pump hose receiving section and a connecting hose receiving section arranged in the corresponding part.

11. The connector of one of the preceding claims, wherein the connector is configured to be arranged in an opening of the pump bed, wherein the connector preferably is configured to be clipped into the opening of the pump bed and/or comprises two clip elements arranged on two sides, in particular two opposite sides, of the connector for clipping to mating connecting surfaces of the pump bed,
wherein preferably a first clip element has a rounded shape and a second clip element has an angled shape, and/or wherein one of the clip elements, preferably a first clip element having a rounded shape, is provided with a connecting element for connecting to an ejection pin of the medical peristaltic pump, wherein the optical data code is preferably arranged on a side of the connector comprising the second clip element, and/or wherein a first clip element is arranged on a side of the first pump hose receiving section and a second clip element is arranged on a side of the second pump hose receiving section,
and/or wherein preferably, the clip elements are integrally formed as parts of a connector part comprising the first and the second fluid path, or wherein preferably, the clip elements are separately formed as at least one separate element connected to a connector part comprising the first and the second fluid path.

12. The connector of one of the preceding claims, wherein a main body of the connector is formed by or comprises at least two tubular fluid path elements comprising the first and a second fluid path, wherein each of the tubular fluid path elements comprises a pump hose receiving section on one end and a connecting hose receiving section on the opposite end,
wherein the connector preferably further comprises a third fluid path connected to the first or the second fluid path and a third connecting hose receiving section associated with the third fluid path for connecting a third connecting hose to the first or the second fluid path via the third fluid path,
wherein the third fluid path is preferably provided by a third tubular fluid path element connected to the first or second tubular fluid path element with one end and comprising the third connecting hose receiving section on its other end, wherein the third tubular fluid path element preferably extends in parallel to the second or first tubular fluid path element, preferably with a distance therebetween.

13. The connector of one of the preceding claims, wherein the connector comprises at least one pressure sensor interface configured to be connected to a pressure transducer, the pressure sensor interface preferably comprising a flexible membrane covering an internal cavity of the connector, the internal cavity fluidly connected to the first or the second fluid path, and/or
wherein the connector is configured as a cassette comprising at least one functional element such as a pressure sensor interface, fluid control element and/or fluid degassing element, wherein optionally, the cassette is configured to positon at least a first and a second pump hose segment in a first and a second pump bed, in particular to position the first and second pump hose segments in a first and a second pump bed in arcuate form,
and/or
wherein the connector is sterile and/or provided in sterilized packaging.

14. A pump hose set comprising:
a connector according to one of the preceding claims and
a pump hose segment connected to the connector and configured for insertion into a pump bed of a medical peristaltic pump, in particular insertion into the pump bed in arcuate form;
wherein the pump hose set preferably further comprises two connecting hose segments connected to the connector, and/or
wherein the pump hose set is preferably a blood tubing set comprising a connector for connecting to a dialyzer provided on one of the connecting hose segments, preferably an outlet connecting hose segment, and/or wherein the pump hose set is preferably sterile and/or provided in sterilized packaging.

15. A medical device, in particular a blood treatment machine, in particular a dialysis machine, comprising a medical peristaltic pump, the medical peristaltic pump comprising
a stator with a pump bed formed therein for receiving a pump hose segment inserted into the pump bed, in particular for receiving the pump hose segment into the pump bed in arcuate form;
an actor, in particular a rotor, for acting on the pump hose segment,
a receiving area for receiving a connector positioning the pump hose segment in the pump bed, and
an optical sensor for reading an optical data code, in particular a barcode, a QR-code or a data matrix code,
**characterized in that**
the optical sensor is configured to read an optical data code arranged on the connector.

16. The medical device of claim 15, wherein the optical sensor is arranged in the receiving area,
wherein preferably the optical sensor is arranged in a bottom surface of the receiving area,
and/or wherein the medical peristaltic pump further comprises at least one ejector for ejecting the connector, the ejector being arranged in the receiving area, wherein preferably the optical sensor and/or a window for the optical sensor and the ejector are arranged with a distance to each other in a bottom surface of the receiving area, and/or
wherein the medical peristaltic pump comprises a lighting unit for lighting the optical data code when the connector is connected to the medical peristaltic pump for reading the optical data code, wherein preferably, the lighting unit emits colored light, wherein further preferably, the lighting unit emits colored light having a complementary color with respect to a base color of the connector;
wherein the medical device comprises a controller programmed to adjust an operation of the medical device based on data read by the optical sensor from the optical data code.

17. A set comprising a medical device according to claim 15 or 16 and a connector according to any one of claims 1 to 10 and/or a pump hose set according to claim 11.

18. A method for manufacturing a pump hose set according to claim 14, comprising the steps of:
- manufacturing the connector,
- connecting the pump hose segment to the connector and preferably connecting the connecting hose segments to the connector and
- sterilizing the tubing set,
the method further comprising the step of
- providing an optical data code on the connector,
wherein preferably, the optical data code is provided on the connector before the step of sterilizing,
wherein further preferably, the optical data code is provided on the connector after connecting the pump hose segment and/or the connecting hose segments to the connector, or
wherein further preferably, the optical data code is provided on the connector before connecting the pump hose segment and/or the connecting hose segments to the connector, wherein preferably, the optical data code provided on the connector is covered during the step of connecting the pump hose segment and/or the connecting hose segments to the connector, in particular by a detachable cover and/or by gripping elements comprising a cover.
